Europäisches Patentamt

European Patent Office

Office européen des brevets

(11) Numéro de publication: **0 277 437 B1**

# FASCICULE DE BREVET EUROPEEN

(45) Date de publication de fascicule du brevet: **03.03.93**  (51) Int. Cl.5: **A61K 39/29**, G01N 33/576

(21) Numéro de dépôt: **87402519.0**

(22) Date de dépôt: **06.11.87**

(54) **Agent viral de l'hépatite NANB, antigène et réactif immunologique.**

(30) Priorité: **07.11.86 FR 8615625**
**11.02.87 FR 8701738**

(43) Date de publication de la demande:
**10.08.88 Bulletin  88/32**

(45) Mention de la délivrance du brevet:
**03.03.93 Bulletin  93/09**

(84) Etats contractants désignés:
**AT BE CH DE ES FR GB GR IT LI LU NL SE**

(56) Documents cités:
EP-A- 0 061 974     EP-A- 0 066 296
EP-A- 0 068 465     EP-A- 0 093 438
EP-A- 0 242 300     WO-A-80/02598
WO-A-82/02774     WO-A-82/03330

BIOLOGICAL ABSTRACTS, vol. 82, no. 6,
1986, page AB-442, résumé no. 53850, Biological Abstracts Inc., Philadelphia, PA, US;
J.L.SARTHOU et al.: "Characterization of an
antigen-antibody system associated with
epidemic non-A, non-B hepatitisin West Africa and experimental transmission of an infectious agent to primates"

BIOLOGICAL ABSTRACTS, vol. 83, no. 12,
1987, page AB-576, résumé no. 119271, Biological Abstracts Inc., Philadelphia, PA, US;
J.PILLOT et al.: "Immunological characterization of a viral agnet involved in epidemic
and sporadic non-A, non-B hepatitis"

(73) Titulaire: **INSTITUT PASTEUR**
**25-28, rue du Docteur Roux**
**F-75724 Paris Cédex 15(FR)**

(72) Inventeur: **Pillot, Jacques**
**14, Chemin de Moulon**
**F-91190 Gif-Sur-Yvette(FR)**

(74) Mandataire: **Orès, Bernard et al**
**Cabinet ORES 6, Avenue de Messine**
**F-75008 Paris (FR)**

## Description

La présente invention est relative à l'isolement et à la caractérisation d'un agent viral impliqué dans l'hépatite non-A non-B tant épidémique que sporadique ou que post-transfusionnelle.

L'hépatite virale peut être causée par le virus de l'hépatite A (HAV), par le virus de l'hépatite B (HBV) et par un nombre inconnu de virus d'hépatite non-A non-B (NANB) non encore identifiés. Actuellement dans les pays développés dans lesquels la prophylaxie de l'HBV est efficace, les NANB sont la cause la plus importante d'hépatite infectieuse conduisant à l'hospitalisation. Cette maladie est considérée comme impliquant au moins trois virus : deux sont transmis par le sang contaminé et les produits de sang contaminé; pour le troisième qui est à l'origine de l'hépatite NANB épidémique, l'Inventeur a pu établir qu'il est également responsable de 50 à 60% des cas d'hépatites nécessitant une hospitalisation, rapportés dans les pays occidentaux et son mode de transmission est inconnu. Dans les pays en voie de développement, le NANB semble également associé à des épidémies à grande échelle liées à un mode de transmission fécal-oral véhiculé par l'eau. Actuellement cinq cas d'épidémies de ce type ont été rapportés depuis 1980 : en Inde, en Sibérie, au Népal, en Algérie et récemment en Côte d'Ivoire. Dans tous ces cas, un taux de mortalité élevé a été observé parmi les femmes enceintes.

De nombreuses recherches ont été effectuées pour isoler un agent causal de l'hépatite NANB et obtenir des préparations antigéniques permettant son diagnostic : on citera, par exemple la Demande de Brevet Européen 68 485 au nom de EISAI CO LTD, la Demande PCT 820 330 et la Demande Européenne 61 974 au nom de C. TREPO, la Demande PCT 82 02774 au nom de BAXTER TRAVENOL LABORATORIES.

Nos connaissances actuelles concernant les virus qui sont à l'origine de l'hépatite NANB sont d'une imprécision décourageante et sont limitées à des données cliniques et épidémiologiques. Des agents filtrables ont nettement été isolés par inoculation de sang ou de produits sanguins provenant de sujets souffrant d'hépatite NANB post-transfusionnelle à des chimpanzés et des produits sanguins infectieux sont disponibles. En ce qui concerne l'hépatite NANB épidémique, très peu d'infections expérimentales ont été réalisées : l'infection s'est avérée transmissible aux ouistitis (Saguinus mystax), aux singes macaques (Macacus cynomolgus) et à un volontaire humain et récemment à des singes verts africains (Cercopithecus aethops et Erythrocebus patas), mais aucune préparation infectieuse définie n'a été proposée.

En ce qui concerne l'hépatite post-transfusionnelle, en dépit de la pléthore de rapports sur des systèmes antigène-anticorps NANB, des modifications ultrastructurales et des particules analogues à des virus, tous potentiellement prometteurs, aucun agent causal ni aucun système ou virus qui remplisse les critères sérologiques acceptés pour une association causale spécifique, n'a été identifié antérieurement aux travaux qui ont abouti à la présente invention. En ce qui concerne l'hépatite épidémique, des particules analogues à des virus, de forme sphérique et de 27 nm de diamètre ont été visualisées dans de très rares fèces et la caractérisation immunologique a été médiocrement réalisée par agglutination au microscope électronique. La réplication virale n'a jamais été obtenue dans des cultures cellulaires pour quelque virus NANB que ce soit.

SARTHOU et al. [Ann. inst. Virol. 137 (2) 225-252 (1986)] décrivent l'infection expérimentale de singes par des extraits fécaux de patients atteints d'hépatite NANB ; les singes infectés produisent un anticorps de classe IgM, qui reconnait un antigène spécifique dans les selles de patients atteints d'hépatite NANB.

La Demande de Brevet français n° 86 05437, au nom de l'INSTITUT PASTEUR, et qui mentionne Monsieur PILLOT comme Inventeur, a pour objet un réactif propre à permettre la détection d'hépatites virales NANB épidémiques et un procédé de diagnostic mettant en oeuvre ce réactif; le réactif conforme à cette Demande de Brevet est constitué par des IgM anti-NANB isolées de sérums de singes infectés artificiellement par des extraits de fèces de patients connus pour être atteints d'hépatite NANB épidémique, lesquelles IgM anti-NANB sont avantageusement fixées sur des supports solides tels que, notamment, des plaques de PVC.

La poursuite de ses recherches à partir de l'invention objet de la Demande de Brevet précitée a permis à l'Inventeur d'atteindre les buts suivants :

1°) Il a infecté des singes dont il a utilisé les anticorps (Ac), ainsi que les Ac de convalescents humains, pour développer un test ELISA pour la détection spécifique d'antigènes (Ag) associés au virus et la détection des Ac anti-viraux;

2°) L'Inventeur a réussi à répliquer le virus dans une lignée continue de cellules hépatocytaires avec une bonne production de matériel antigénique;

3°) Il est parvenu à visualiser des particules virales dans des préparations purifiées d'échantillons de fèces et dans des lignées d'hépatocytes infectées;

4°) Il a pu établir l'implication du même agent causal dans l'hépatite virale NANB aussi bien épidémique que sporadique.

5°) En considérant le rôle de l'agent NANB épidémique dans l'hépatite sporadique, l'Inventeur a été en mesure de vérifier que ledit agent peut être non seulement véhiculé par l'eau, mais être également transmis par d'autres voies que l'eau et notamment par la voie sanguine transfusionnelle.

La présente invention s'est donné pour but l'identification et la caractérisation d'un nouvel agent viral, ainsi que d'un antigène (Ag) NANB associé à ce nouvel agent viral et une méthode de diagnostic d'hépatite NANB aussi bien épidémique que sporadique et que post-transfusionnelle, par détection de l'Ag associé au virus de NANB, en utilisant les anticorps (Ac) qui font l'objet de la Demande de Brevet INSTITUT PASTEUR précitée ou par recherche des Ac du malade, en utilisant le virus ou ses fractions adsorbé sur un support adéquat.

La présente invention a également pour but de pourvoir à des réactifs de diagnostic immunologiques utilisables dans la méthode de diagnostic de l'hépatite NANB conforme à la présente invention, ainsi qu'à un kit prêt à l'emploi pour la réalisation de ladite méthode de diagnostic.

La présente invention a pour objet un agent viral purifié associé à l'hépatite virale NANB épidémique, sporadique et post-transfusionnelle, caractérisé en ce qu'il est susceptible d'être obtenu à partir de fèces de patients atteints d'hépatite NANB et qu'il se présente sous la forme de particules virales sphériques de deux types principaux, associées à un même antigène viral :

a) des particules sphériques d'environ 75 nm de diamètre, dont la densité sur un gradient de CsCl est comprise entre 1,28 et 1,32 g/cm$^3$, et qui sédimentent dans les fractions d'un gradient de saccharose contenant entre 40 et 41,5% (p/v) de saccharose,

b) des particules sphériques de 25 à 30 nm de diamètre, dont la densité sur un gradient de CsCl est comprise entre 1,12 et 1,19 g/cm$^3$, et qui sédimentent dans les fractions d'un gradient de saccharose contenant entre 26 et 28% (p/v) de saccharose.

La présente invention a, de plus, pour objet un procédé d'isolement d'un agent viral impliqué dans l'hépatite virale NANB épidémique, sporadique et post transfusionnelle, qui est caractérisé en ce que l'on procède à la centrifugation, sur un gradient de CsCl ou sur un gradient de saccharose, d'un extrait biologique contenant ledit agent viral, et en ce qu'on recueille ledit agent dans les fractions du gradient de CsCl dont la densité est comprise entre 1,29 et 1,32 g/cm$^3$ dans les fractions du gradient de saccharose contenant entre 40 et 41,5% (p/v) de saccharose, sous forme de particules de 75 nm de diamètre environ, et/ou dans les fractions du gradient de CsCl dont la densité est comprise entre 1,12 et 1,19 g/cm$^3$ ou du gradient de saccharose contenant entre 26 et 28% (p/v) de saccharose, sous forme de particules de 25 à 30 nm de diamètre.

Selon un mode de mise en oeuvre du procédé d'isolement de l'agent viral susdit, le traitement de centrifugation est avantageusement constitué par une opération de centrifugation à 5000 - 8000 g environ pendant 10 minutes environ.

Selon un autre mode de mise en oeuvre du procédé d'isolement de l'agent viral susdit, préalablement aux opérations de centrifugation et de filtration susdites, l'extrait de fèces est soumis à un traitement par ultra-sons ou à un traitement de dissociation chimique.

La présente invention a également pour objet un autre procédé d'obtention de l'agent viral susdit, qui est caractérisé en ce que des hépatocytes ou des lignées cellulaires continues d'hépatocytes sont inoculés par un extrait de fèces d'un patient atteint d'hépatite NANB ou par un extrait de foie d'un singe (de préférence Saimiri) inoculé par un extrait de fèces de patients souffrant d'hépatite NANB, et cultivés jusqu'à confluence de la culture, à partir de laquelle est réalisée une sous-culture, qui est centrifugée après plusieurs passages, pour recueillir l'agent viral recherché.

Selon un mode de mise en oeuvre avantageux de ce procédé, le culot de centrifugation est repris par un tampon approprié pour être soumis à un traitement par ultrasons qui libère dans le surgangeant, l'Ag NANB.

Des souches de l'agent viral conforme à l'invention, isolées comme décrit dans ce qui précède et ci-après, et caractérisées comme indiqué plus loin, ont été déposées auprès de la COLLECTION NATIONALE DE CULTURE DE MICROORGANISMES tenue par l'INSTITUT PASTEUR, en date du 30 Octobre 1986, à savoir :

- une souche d'agent viral isolé de fèces d'un malade atteint d'hépatite NANB sporadique, dite "Souche Clamart", qui a été affectée du Numéro de dépôt : I-617,
- une souche d'un agent viral isolé de fèces d'un malade atteint d'hépatite NANB épidémique, dite "Souche ivoirienne", qui a été affectée du Numéro de dépôt : I-616.

L'Invention a également pour objet un procédé de détection des anticorps présents dans le sang du malade ou du convalescent ou dans des produits utilisés en transfusion sanguine ou dans des fractions dérivées de sang, ayant été en contact avec le virus agent causal de l'hépatite non A non B, tel que défini selon la présente invention, dont les souches I-617 et I-616 et celles présentant une réaction immunologique croisée avec les deux souches de référence, selon ce procédé, le sérum du malade ou du patient convalescent est mis en contact avec la préparation virale purifiée (ou avec une fraction de celle-ci) telle que recueillie dans les conditions mentionnées ci-dessus, après centrifugation et isolement de la bande correspondant à une densité de 1,30.

Ladite préparation virale est fixée, par exemple sur les parois d'une microplaque selon les techniques connues.

Le complexe Ag/Ac est révélé par une préparation d'anti-immunoglobulines humaines marquées par un marqueur adéquat, par exemple une enzyme.

Un système analogue peut être utilisé pour caractériser une activité anticorps dans les IgM d'un patient en vue d'un diagnostic sérologique très précoce de la maladie, en utilisant des anti-IgM humaines, ou mieux en captant les IgM d'un sérum en les faisant réagir avec le virus ou ses fractions et en caractérisant l'Ag fixé sur l'IgM du malade.

Le procédé de diagnostic in vitro de la présence du virus de l'hépatite NANB conforme à la présente invention consiste donc à mettre en contact un sérum ou un autre milieu biologique d'un patient dont on cherche à établir le diagnostic, avec au moins une des protéines ou des glycoprotéines du virus NANB ou avec un lysat viral ou avec un extrait viral, puis à détecter la réaction immunologique par la méthode ELISA ou par une autre méthode immunoenzymatique ou une méthode par immunofluorescence, qui peut mesurer directement ou indirectement l'immunofluorescence ou la réaction immunoenzymatique.

C'est la raison pour laquelle la présente invention englobe également les extraits viraux marqués, qu'ils soient marqués par une enzyme, par fluorescence ou qu'ils soient marqués par un radioisotope.

Les méthodes d'immunodétection comprennent, comme on le sait :
- le dépôt de quantités d'extrait spécifique ou des protéines virales conformes à l'invention, dans ou sur un support tel que les puits d'une microplaque de titration ;
- l'introduction de dilutions croissantes du sérum à diagnostiquer sur ledit support, tel que lesdits puits ;
- l'incubation du support tel que la microplaque susdite ;
- le lavage soigneux du support tel que la microplaque susdite à l'aide d'un tampon convenable ;
- l'introduction d'anticorps anti-immunoglobuline humaine marqués spécifiques dans ou sur le support tel que les puits de la microplaque susdite, le marquage étant réalisé par une enzyme choisie parmi celles capables d'hydrolyser un substrat de telle manière que l'absorption de radiations par ce dernier soit modifiée au moins dans une bande spécifique de longueurs d'ondes, et
- la détection, de préférence de manière comparative, par rapport à un témoin, de la quantité de substrat hydrolysé, à la fois par la mesure du produit biologique dangereux et par la présence effective de la maladie.

La présente invention a également pour objet un kit - ou ensemble - prêt à l'emploi pour la réalisation du procédé de diagnostic (recherche de l'anticorps ou du virus NANB) conforme à l'invention, défini plus haut, lequel kit est caractérisé en ce qu'il comprend :
- un extrait ou une fraction purifiée de l'agent viral conforme à l'invention, lequel extrait ou fraction est marqué, par exemple par un radioisotope, une enzyme ou par immunofluorescence ;
- des anti-immunoglobulines humaines ou une protéine A éventuellement fixées sur un support insoluble dans l'eau tel que sphères d'agarose ou de latex, magnétiques ou non ;
- des tampons et, si nécessaire, des substrats pour visualiser les marquages.

La caractérisation de certains des antigènes qui entrent dans la composition de l'agent viral isolé conformément à la présente invention a été réalisée dans un premier temps sur la souche Clamart, en procédant comme décrit ci-après.

La souche Clamart a été isolée à partir de selles d'un patient atteint d'hépatite NANB sporadique.

Le surnageant desdites selles a été centrifugé en gradient de chlorure de césium dont la bande contenant l'antigène viral purifié comme défini plus haut a été recueillie et sa densité déterminée comme étant de 1,30.

Les épitopes reconnus dans le text de diagnostic défini plus haut, sont portés par ces antigènes, qui peuvnet à ce titre, être utilisés dans ledit test.

La recherche d'anticorps à l'aide de cette méthode de détection peut être effectuée dans le sang de malades ou de convalescents, dans les produits utilisés dans les transfusions sanguines et dans les fractions dérivées du sang de personnes ayant été exposées à l'agent causal de l'hépatite NANB tel que défini plus haut, parmi lesquelles les souches I-617 et I-616 et celles présentant une réaction immunologique croisée avec ces deux souches de référence, notamment la souche isolée de patients atteints d'hépatite NANB post-tranfusionnelle, dont les propriétés immunologiques sont identiques à celles de la souche I-617, et qui est identifiée comme souche virale "H-TEST" déposée le 5 Février 1987 sous le N° 87.02.05.02 auprès de la Collection ECACC (Grande-Bretagne).

Bien que les données cliniques et épidémiologiques aient pu suggérer que la forme épidémique de l'hépatite NANB présentée par des patients atteints d'hépatite NANB épidémique est due à un virus différent de celui qui est à l'origine de l'hépatite NANB post-transfusionnelle transmise par injection parentérale, conformément à la présente invention l'Inventeur a pu isoler de patients atteints d'hépatite NANB post-transfusionnelle une nouvelle souche de virus NANB dont les propriétés immunologiques sont pratiquement identiques à celles des souches déposées le 30 Octobre 1986 sous les N° I-616 et I-617 auprès de la CNCM de l'Institut Pasteur. Des tests sérologiques très sensibles de présence de virus HAV et HBV ont permis d'exclure ces deux virus en tant qu'agents éthiologiques et d'exclure, de même, d'autre virus hépatotropes tels que le cytomégalovirus, le virus d'Epstein-Barr et le virus de la fièvre jaune.

La présente invention a en outre pour objet un nouvel agent viral purifié impliqué dans les formes sporadique, épidémique et post-transfusionnelle de l'hépatite NANB. Ce virus se distingue des virus HAV et HBV en ce qui concerne l'homologie antigénique de ses protéines en tant que matériel génétique.

Les compositions préparées conformément à la présente invention (antigènes viraux purifiés, protéines recombinantes obtenues par l'expression du virus NANB dans des cellules procaryotes ou eucaryotes ou peptides synthétiques déduits de la séquence du génome) peuvent être utilisées pour le diagnostic de l'hépatite NANB ou pour la vaccination propre à induire la synthèse d'une réponse immune protectrice après administration à l'hôte.

La présente invention englobe toutes les compositions de ce type contenant un antigène présentant des propriétés immunologiques équivalentes à celles du virus NANB Clamart (CNCM I-617) ou Côte d'Ivoire (CNCM I-616). En effet, deux protéines ou antigènes sont considérés comme équivalents dans le cadre de la présente invention lorsqu'ils sont capables d'être reconnus par les mêmes anticorps. Les produits exprimés par des séquences correspondantes du matériel génétique codant des séquences génétiques correspondantes sont, par suite, compris dans le cadre de tels antigènes équivalents.

La présente invention englobe également les sérums pouvant être produits à partir d'animaux par inoculation à ces derniers de virus NANB à l'aide des compositions précitées. En particulier, la présente invention englobe les anticorps polyclonaux spécifiquement dirigés contre chacun des antigènes du virus NANB. Elle englobe également les anticorps monoclonaux susceptibles d'ête obtenus par des méthodes appropriées et dirigées plus spécifiquement contre les antigènes du virus NANB.

Conformément à la présente invention, l'utilisation du virus ou de son lysat ou d'une fraction de ce lysat peut être envisagée pour la préparation d'anticorps monoclonaux et le lysat viral ou les fraction de celui-ci ou les protéines purifiées à partir de ce virus, du lysat ou de ses fractions, peuvent être utilisés pour la recherche d'anticorps dans le sérum des patients par mise en oeuvre de tests classiques de type RIPA, ELISA ou Western blot (immunoempreinte).

Ces anticorps polyclonaux et monoclonaux peuvent être utilisés dans un grand nombre d'applications au nombre desquelles on peut citer la neutralisation de l'antigène correspondant ou du virus total. Ils peuvent être utilisés pour détecter des antigènes viraux dans des préparations biologiques ou pour purifier des antigènes correspondants.

La présente invention englobe également tout virus de l'hépatite NANB équivalent présentant les mêmes caractéristiques immunologiques. Les travaux menés par l'Inventeur ont montré la relation immunologique entre les matériels antigéniques isolés d'extraits de fèces de malades atteints respectivement d'hépatite NANB épidémique, sporadique et post-transfusionnelle.

C'est ainsi que la souche de type Clamart (déposée sous le N° I-617 auprès de a CNCM et également déposée auprès de la Collection ECACC sous le N° 87.02.05.02) a été isolée à partir des selles d'une malade qui présentait une hépatite aigüe NANB dûment caractérisée comme hépatite NANB transfusionnelle. Les selles contenaient l'antigène conforme à l'invention, également caractérisé , conformément à l'invention, dans les hépatites épidémiques (Côte d'Ivoire)et sporadiques (France).

Un antigène associé au virus de l'hépatite NANB a été caractérisé pendant 15 jours à la phase aigüe de la maladie. D'autres cas similaires ont été observés avec caractérisation du même antigène dans les selles et isolement d'un virus présentant les caractéristiques immunologiques identiques aux virus I-616 et I-617.

Outre les dispositions qui précèdent, l'invention comprend encore d'autres dispositions, qui ressortiront de la description qui va suivre.

L'invention sera mieux comprise à l'aide du complément de description qui va suivre, qui se réfère à des exemples de mise en oeuvre du procédé objet de la présente invention.

Il doit être bien entendu, toutefois, que ces exemples de mise en oeuvre sont donnés uniquement à titre d'illustration de l'objet de l'invention, dont ils ne constituent en aucune manière une limitation.

Au cours d'une première étape, deux singes Cercopithecus aethops et un singe Erythrocebus patas ont été inoculés par voie orale par 2 ml d'un mélange d'un extrait aqueux à 10% de fèces de patients ivoiriens présumés atteints d'hépatite NANB épidémique, collectées au cours des premiers jours ayant suivi l'apparition des symptômes. Le mélange avait été préalablement centrifugé et filtré à travers des filtres Millipore® de 0,22 um pour éliminer les bactéries et les parasites. Les échantillons de sérum et les échantillons de fèces de ces singes ont été collectés d'une part avant l'administration de l'extrait de fèces et d'autre part le vingt-septième jour après l'inoculation. Les fonctions hépatiques de ces singes n'ont pas été étudiées; ces animaux ne présentaient pas de manifestations cliniques évidentes. Un singe Saimiri a également été inoculé dans les mêmes conditions par 0,5 ml d'un extrait de fèces provenant d'un patient ivoirien différent de ceux utilisés pour les singes verts d'Afrique. Un second singe Saimiri qui avait été inoculé trois mois auparavant par une préparation fécale de virus d'hépatite A et séroconverti à l'aide d'anticorps IgM anti-HAV au jour 29, a été inoculé par voie intraveineuse par 0,5 ml du même échantillon de fèces filtré. Les deux singes Saimiri ont été saignés deux fois par semaine pour effectuer des examens biochimiques et sérologiques et leurs fèces ont été examinées quotidiennement. Les deux singes Saimiri sont morts au jour 28, sans avoir présenté de symptômes particuliers. Aucun des singes n'avait présenté de preuve biochimique convaincante d'un dysfonctionnement hépatique et l'examen histologique effectué post-mortem a montré une congestion du foie sans lésion du parenchyme. Les sérums de ces cinq singes ont été fractionnés dans du tampon Tris NaCl 0,1M, pH 8, sur du "Sephacryl® S 300" et les fractions contenant des IgM et des IgG ont été collectées. Elles ont été utilisées en tant qu'anticorps récepteurs dans un test immunoenzymatique. L'IgM a été choisie pour sa capacité de capture élevée et pour empêcher les réactions faussement positives dues à un facteur rhumatoïde ou analogue au facteur rhumatoïde, qui a été reconnu comme stimulant l'antigène NANB.

En fait, on pourrait également utiliser l'IgG bien que son bruit de fond soit un peu plus élevé que celui de l'IgM et sa spécificité déficiente pour certaines préparations.

Pour révéler la fixation d'antigène de fèces sur l'anticorps en phase solide, des IgG provenant de sérums de convalescents ont été fractionnées par chromatographie sur DEAE-trisacryl, après précipitation par du $(NH_4)_2SO_4$. Les IgG ont été conjuguées à la $\beta$-galactosidase ou à la peroxydase. Des IgG marquées ont été utilisées pour la détection de l'antigène : une préparation d'IgG provenant d'un patient ivoirien convalescent d'une hépatite NANB épidémique; une préparation d'IgG prélevée chez un patient algérien guéri, au cours de l'épidémie de Medea en 1981-1982; enfin l'IgG d'un singe Cercopithèque inoculé expérimentalement. Les préparations d'IgG des trois origines ont donné des résultats équivalents. Pour chaque singe, on a utilisé de l'IgM de pré-inoculation en phase solide comme témoin de spécificité d'IgM de post-inoculation. De l'IgG humaine marquée non-immune a été utilisée comme témoin de marquage d'Ag. Les fèces de singes inoculés ont été examinées pour établir la présence d'un Ag présumé NANB dans le test ELISA. Une activité antigénique a été détectée dans les fèces des deux singes Saimiri (cf. Tableau 1). L'application d'un traitement par ultra-sons a permis d'augmenter la quantité d'antigène détecté et a augmenté la sensibilité de la méthode. L'Ag est apparu dès le quatrième jour après l'inoculation et a persisté jusqu'au treizième jour.

Le Tableau 1 montre l'Ag NANB détecté dans les fèces de singes infectés expérimentalement par des extraits de fèces provenant de patients atteints de NANBH épidémique.

La détection a été réalisée comme suit, en mettant en oeuvre la technique ELISA : des immunoplaques NUNC® ont été revêtues de 100 $\mu$l de 50 $\mu$g/ml de fraction d'IgM, échantillonnées 27 jours après inoculation, dans une solution physiologique de tampon phosphate (PBS) pendant une heure à 37°C et une nuit à 4°C. Après plusieurs lavages, les plaques ont été recouvertes de 200 $\mu$l de PBS contenant 1% de sérum-albumine bovine et 0,1% de Tween® 20, pendant trois heures à 37°C. Après lavage, 100 $\mu$l des extraits de fèces (1/10 v/v) filtrés sur un élément de porosité 0,22 $\mu$m sont incubés sur les IgM du revêtement, à 37°C pendant une heure, puis une nuit à 4°C. Après plusieurs lavages, 100 $\mu$l d'IgG de singe immun marquée à la peroxydase à 5 mcg/ml dans 1% de BSA + 1% de Tween® 20 dans du tampon Tris sodique, pH 7,6 (TNB) contenant 0,1 % de $NaN_3$, ont été utilisés pour la détection de l'Ag. Au bout d'une heure d'incubation à 37°C et plusieurs lavages, la réaction a été développée à l'aide de 100 $\mu$l d'orthophénylène-diamine dans 0,005 M de tampon citrique pH 5,6, avec 1 $\mu$l/ml $H_2O_2$. Les résultats obtenus sont exprimés par le rapport entre l'échantillon étudié et la moyenne de cinq échantillons négatifs (P/N). Le bruit de fond des échantillons négatifs a été de l'ordre de 0,04-0,06 en densité optique (DO) à 492 nm. Il est plus élevé si le $NaN_3$ a été omis, mais, dans ce cas, des quantités de réactifs plus faibles doivent être mises en réaction. Les échantillons dont le rapport P/N 2,1, ont été considérés comme positifs. Les fèces (1/10 poids/poids) qui ont été examinées étaient de deux types : celles ayant subi un traitement aux ultra-sons et celles n'ayant pas subi un tel traitement; le traitement aux ultra-sons appliqué est de trois minutes à 4°C, avec une intensité de sortie de 4 avec l'appareil de BRENSON pour des volumes de 2 à 3 ml avant centrifugation et filtration à travers un élément de porosité de 0,22 $\mu$m. Les résultats positifs sont soulignés dans le Tableau 1. Les fèces d'un singe Saimiri ayant reçu de l'extrait de fèces d'un patient atteint d'une maladie du foie non-infectieuse ont servi de témoin et ont constamment donné des résultats négatifs.

TABLEAU 1

| Nombre de jours après inoculation | P/N sans traitement par ultra-sons | | P/N avec traitement par ultra-sons | |
|---|---|---|---|---|
| | Singe N° 1 (inoculation par voie intraveineuse) | Singe N° 2 (inoculation par voie orale) | Singe N° 1 | Singe N° 2 |
| 0 | 1,15 | 1,14 | 1,18 | 1,07 |
| 4 | 2,47 | 1,60 | 5,10 | 0,79 |
| 7 | 6,48 | 3,28 | 8,78 | 5,52 |
| 9 | 3,64 | 2,24 | 4,72 | 5,82 |
| 11 | 1,12 | 1,40 | 1,95 | 5,20 |
| 13 | 1,30 | 1,46 | 2,40 | 0,80 |
| 15 | 1,51 | 1,68 | 0,93 | 0,90 |
| 19 | 1,53 | 1,51 | 1,20 | 0,80 |

Une deuxième étape a consisté à étudier des fèces de patients atteints d'une hépatite NANB aigüe présumée, aussi bien sous sa forme épidémique (patients ivoiriens) que sous sa forme sporadique (patients français). Le diagnostic de l'hépatite NANB a été établi conformément aux critères immunologiques classiques des trois types d'infections par des virus d'hépatite (A,B, delta) : présence ou absence d'IgM et d'IgG anti-HAV pour l'infection par le virus A; HBsAg, anti-HBc et IgM anti-HBc, anti-HBs, pour l'infection par HBV; les Ag delta et anti-delta pour l'infection par HDV, le cas échéant. Pour les patients ivoiriens, les marqueurs de la fièvre jaune ont été recherchés. On a également recherché chez tous ces patients l'infection par le cytomégalovirus par immunocapture d'anticorps IgM et l'infection par le virus d'Epstein-Barr, par détection d'IgM anti-VCA dans les taches immunofluorescentes. L'Ag associé à la NANB a été détecté aussi bien dans les cas épidémiques que dans les cas sporadiques (cf. Tableau 2). Il n'a pas été détecté d'Ag dans 90 extraits témoins de patients européens présentant d'autres troubles gastro-intestinaux. Dans la plupart des cas, ces résultats ont été confirmés plusieurs fois avec différentes IgM ou IgG en phase solide et avec différentes IgG marquées. Les mêmes échantillons positifs ont été étudiés avec l'IgM pré-immune recouvrant une plaque et il n'a pas été détecté d'antigène. Il en résulte que l'Ag détecté paraît étroitement lié au virus NANB.

Le Tableau 2 ci-après illustre la détection d'Ag NANB dans les fèces de patients atteints d'hépatite NANB épidémique (de Côte d'Ivoire) et sporadique (de France). 90 fèces de patients suspectés d'infections gastro-intestinales ont été utilisées comme témoins. Tous les échantillons présumés NANB et 17 des 90 témoins ont été soumis à un traitement par ultra-sons. De l'anticorps (Ac) humain marqué à la peroxydase ou la $\beta$-galactosidase, a été utilisé comme traceur d'Ag.

TABLEAU 2

|  | Echantillons de fèces de Côte d'Ivoire : cas épidémiques | Echantillons de fèces de France : cas sporadiques | Témoins de France |
|---|---|---|---|
| AG NANB positif | 17 | 7 | 0 |
| AG NANB négatif | 44 | 10 | 90 |

Pour vérifier que l'Ag détecté est effectivement étroitement lié au virus NANB, au cours d'une troisième étape, des lignées cellulaires continues d'origine hépatocytiques, PLC/PRF 5 et HEP G2, et des cellules normales humaines fibroblastiques MRC 5, ont été inoculées dans du milieu de DULBECCO contenant 10% de sérum de veau foetal, par un extrait de fèces d'un malade atteint d'hépatite NANB. Après confluence de la culture, une sous-culture a été réalisée après addition d'EDTA-trypsine. Dans certains cas, des substances toxiques présentes dans les fèces provoquent la mort des cellules précocement lors de la première semaine, tandis que d'autres fèces non toxiques pour les lignées cellulaires, induisent la mort des cellules après plusieurs passages. Cet effet léthal spécifique a été observé uniquement avec la lignée cellulaire PLC/PRF 5 mais ne l'a pas été avec la lignée cellulaire HEP G2 et les cellules MRC 5. Après traitement des cellules aux ultra-sons et filtration sur porosité 0,22 $\mu$m, de l'Ag NANB a été détecté dans le surnageant de PLC/PRF 5 infectées mais pas dans le surnageant des HEP G2 infectées dans les mêmes conditions.

Pour des extraits de foie de singes inoculés, on n'a pas observé de toxicité cellulaire après inoculation et les cellules PLC/PRF 5 ont présenté une positivité antigénique de la même manière que dans le cas d'échantillons de fèces (cf. Tableau 3).

Le Tableau 3 illustre la détection d'Ag NANB dans la lignée cellulaire d'hépatome inoculée par un extrait de fèces d'un patient ivoirien souffrant d'hépatite NANB épidémique et par un extrait de foie d'un singe Saimiri infecté (N° 1).

L'extrait de foie a été préparé dans du PBS (10% poids/poids) et homogénéisé à l'aide de l'"Ultraturax"® Après centrifugation, le culot a été remis en suspension dans 4 fois son volume de TNB et soumis à un traitement aux ultra-sons en présence de 0,005 mg/ml de chlorure de phénylméthyl-sulfonide (PMSF).

Un extrait de foie d'un singe Saimiri inoculé par un extrait de fèces de patients souffrant d'une maladie de foie non-infectieuse a servi de témoin.

Après trois passages, les cultures cellulaires inoculées ont été centrifugées. Le culot a été repris dans du TNB et après addition de PMSF, les cellules ont été soumises à un traitement aux ultra-sons. Les chiffres indiquent le rapport P/N.

TABLEAU 3

| Lignée cellulaire d'hépatome | Cellules inoculées par de l'extrait de fèces de patient ivoirien | Cellules inoculées par de l'extrait de foie de singe Saimiri infecté | Cellules inoculées par de l'extrait de foie de singe Saimiri témoin | Cellules non-inoculées |
|---|---|---|---|---|
| PLC/PRF 5 | 8,19 * | 4,39 | 2 | 1,01 |
| HEP G2 | 1,62 | 1,14 | 2 | 1,07 |

* après dilution à 1/100 P/N : 6,29.

Il semble ressortir des essais préliminaires de marquage par immunofluorescence qu'une cellule sur 50 environ, seulement, soit infectée, dans les conditions expérimentales.

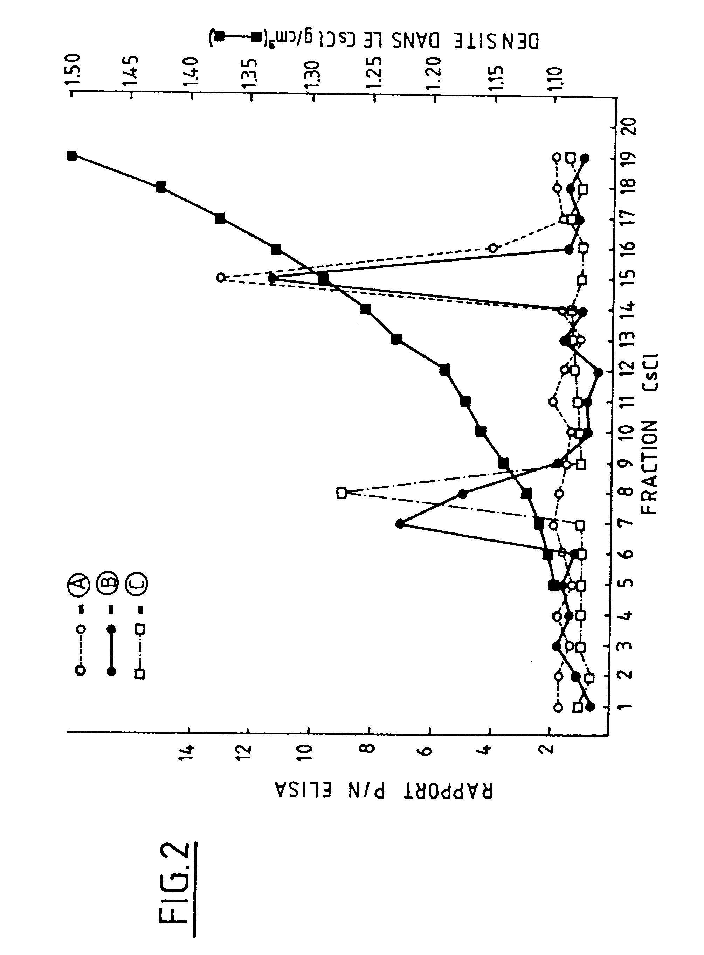

Une quatrième étape a eu pour objet de purifier l'antigène associé au virus, provenant d'échantillons de fèces de différentes origines. Trois profils de répartition d'antigène ont été observés et sont représentés à la figure 1 annexée qui montre que dans des échantillons fraîchement utilisés, l'activité antigénique apparaît sous la forme d'une bande à une densité de 1,29-1,32 g/cm$^3$. Certains des échantillons de fèces conservés à + 4°C pendant quelques mois, ou des échantillons frais ayant subi un traitement par ultra-sons, n'ont présenté d'activité antigénique qu'à une densité de 1,12-1,19 g/cm$^3$, tandis que d'autres présentaient une activité aux deux densités.

La figure 1 représente la répartition de l'antigène dans le gradient de CsCl après ultracentrifugation isopycnique, obtenue en procédant comme suit :

3 ml d'échantillons de fèces (10 % dans RPMI) ont été fractionnés après filtration à travers un élément filtrant de 0,22 $\mu$m de porosité, dans un gradient de CsCl de densité comprise entre 1,1 et 1,6 g/ml. La centrifugation isopycnique (48 heures) à 78000 g a été effectuée dans des tubes de 12 ml (1,2,3,2, 0,5, 0,5 ml de CsCl de densité 1,1, 1,2, 1,3, 1,4, 1,5 et 1,6 respectivement). 0,6 ml de fractions ont été collectés et testés pour déterminer la présence d'antigène NANB après deux dilutions.

Dans la figure 1 :

A désigne les fèces fraîches

B et C des fèces conservées ou traitées aux ultra-sons.

Du matériel antigénique à faible densité apparaissant dans ces conditions est supposé résulter d'un effet de décomposition par des enzymes contenues dans les fèces ou par un traitement physique. Les fractions de CsCl à antigénicité positive ont été soumises à une ultracentrifugation dans un gradient de saccharose variant de 10 à 60 % poids/volume et les fractions réactives ont été étudiées à l'ultramicroscope. L'antigène NANB a été trouvé à 40-41,5 % de saccharose et de façon prédominante à 26-28 % de saccharose. Sept préparations obtenues à partir de 9 échantillons à antigénicité NANB positive, comportaient des particules virales : une provenant d'un singe Saimiri inoculé (le second singe n'a pas été examiné), deux provenant de deux des trois patients atteints d'hépatite NANB épidémique aigüe (Côte d'Ivoire), quatre provenant de quatre des cinq patients atteints d'hépatite NANB sporadique (France). Deux types de particules virales sphériques ont été observées : des particules de grandes dimensions , de l'ordre de 75 nm de diamètre, qui présentent une structure à double membrane, et des particules de dimensions plus petites, homogènes, de 25 à 30 nm de diamètre environ, sans aucune structure interne définie. Une structure tubulaire de 15 nm de diamètre (ou de largeur) et dont la longueur peut atteindre 1 $\mu$m, plus ou moins fixée aux particules, a été observée et est représentée à la figure 2 annexée. Certaines images donnent l'impression que des tubules partent de l'enveloppe des particules virales de grandes dimensions. Les particules les plus caractéristiques ont été observées dans des échantillons frais n'ayant pas subi de traitement par ultra-sons. Des particules sphériques de grandes dimensions ont pu être observées pour la plupart dans des fractions de densité comprise entre 1,29 et 1,32 g/cm$^3$ et dans la fraction de 40-41,5 % de saccharose du gradient de saccharose, tandis qu'on n'a pu observer que des particules de dimensions moindres dans les fractions de CsCl présentant une densité comprise entre 1,12 et 1,19 g/cm$^3$ et dans la fraction du gradient de saccharose à 26-28 % de saccharose. Il n'a pas encore pu être établi si les particules les plus petites et de densité plus basse représentent une forme de libération du virus de départ. Six échantillons de fèces à antigénicité négative ont également été étudiés simultanément de la même manière pour servir de témoins. On n'a trouvé dans aucune de ces préparations une structure ayant l'aspect de particules.

Une dernière étape a permis de mettre en évidence que la détection d'Ag NANB dans les fèces démontre que le virus NANB épidémique est fréquemment impliqué dans l'hépatite NANB sporadique. De l'Ag NANB associé au virus a été détecté dans sept échantillons de fèces parmi 17 patients examinés atteints d'hépatite NANB sporadique aiguë (cf. figure 2). Des examens sérologiques ont confirmé ces résultats : parmi huit patients dont on disposait des sérums, sept d'entre eux présentaient un sérum avec l'anticorps anti-NANB.

La figure 2 illustre la caractérisation de particules virales au microscope électronique dans les fèces de singes infectés par NANB et de patients atteints d'hépatite NANB.

Après ultracentrifugation isopycnique comme décrit en relation avec la figure 1, les fractions positives ont été mélangées, puis dialysées contre du TNB, puis elles ont été soumises à une ultracentrifugation zonale dans un gradient de saccharose (10-60 % poids/poids) à 78000 g pendant 5 heures. Les fractions positives ont été mélangées, dialysées, concentrées et examinées au microscope électronique. Une goutte de préparation virale a été étalée sur une grille de 200 mesh revêtue de carbone et après absorption sur papier-filtre, une goutte de 2 % d'acétate d'uranyle dans de l'eau distillée a été ajoutée. Au bout de deux minutes, la grille a été essuyée avec du papier-filtre et étudiée au microscope JEM 100 C x II.

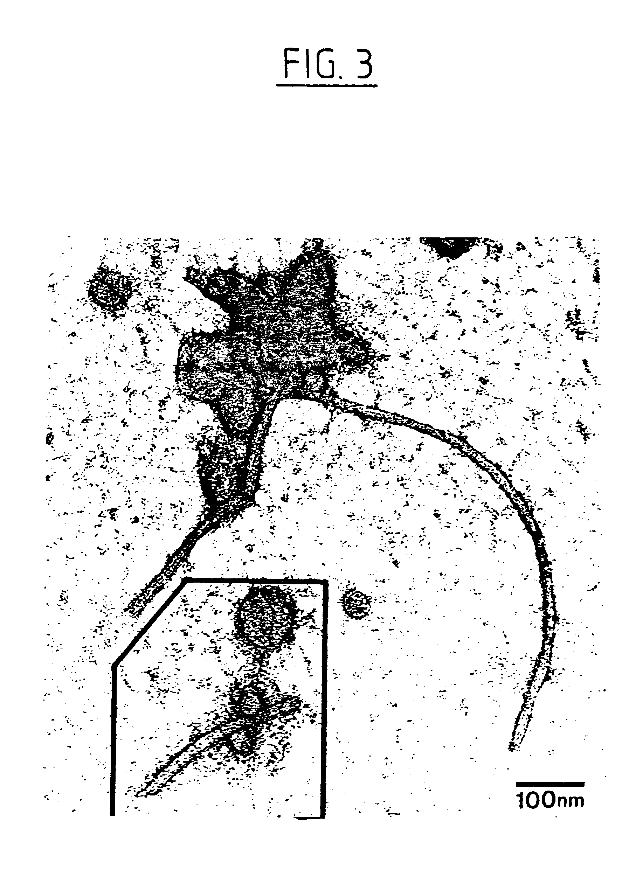

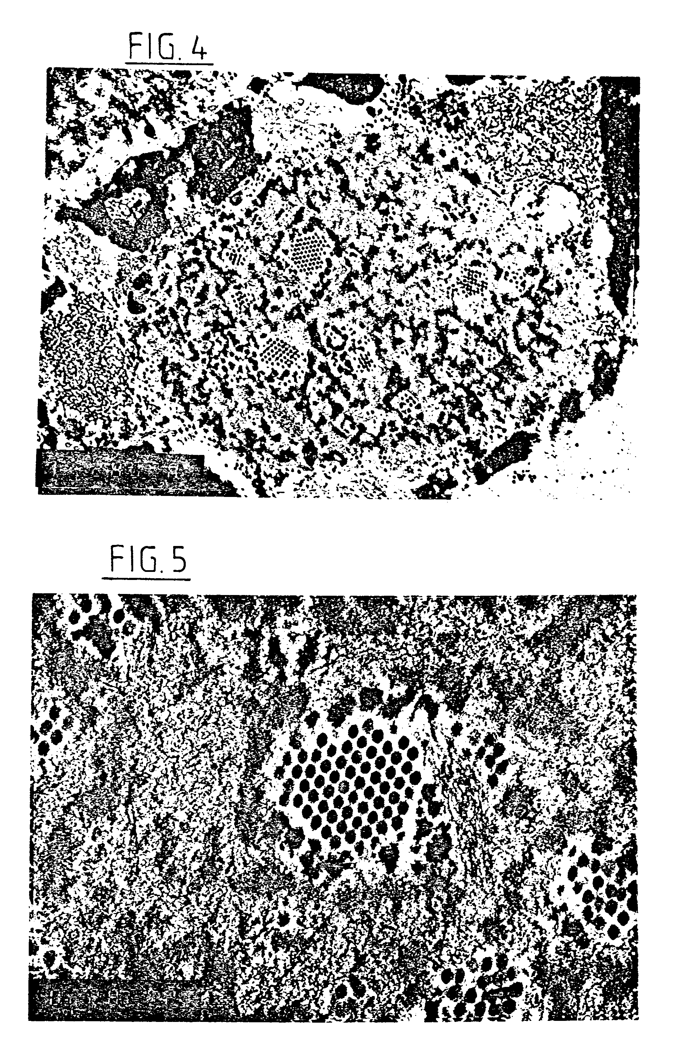

Les figures 3 et 4 annexées représentent des coupes vues au microscope électronique après coloration par l'acétate d'uranyle de cellules PLC/PRF 5 inoculées avec un extrait de selles d'un patient souffrant d'hépatite non A non B chez lequel l'Ag a été détecté dans ses selles selon les techniques décrites plus haut.

Les particules virales sont retrouvées dans le noyau et le cytoplasme sous forme de "réseaux cristallins" ; le diamètre de la particule est très constant : 74 à 75 nm. La figure 3 est une vue à grossissement X16000 et la figure 4 une vue à grossissement 54000.

Comme les épidémies d'hépatite NANB sont habituellement observées dans les pays en voie de développement, il a paru intéressant d'examiner si les mêmes virus jouent, dans ces pays, un rôle dans les cas sporadiques. C'est pourquoi 28 sérums de patients marocains souffrant d'hépatite NANB aiguë sporadique ont été examinés pour déterminer s'ils contiennent des anticorps vis-à-vis du virus NANB épidémique. 68 % des patients se sont avérés présenter des anticorps anti-virus NANB épidémique tandis que les mêmes anticorps n'ont été détectés que dans 10 % de sérums de donneurs de sang, dans le même pays.

Le Tableau 4 ci-après illustre la détection d'anticorps spécifiques vis-à-vis de l'antigène NANB dans des sérums de patients souffrant d'hépatite NANB sporadique. La présence d'anticorps a été démontrée par inhibition de la réaction dans les conditions décrites dans le Tableau 1 ci-dessus. La réaction a été réalisée en incubant 50 $\mu$l d'un extrait de référence provenant de fèces de singe infecté, pendant une heure à 37°C et une nuit à 4°C, avec 50 $\mu$l du sérum à examiner. Toutes les autres étapes de la réaction ont été réalisées de la même manière que pour la détection de l'Ag. Le rapport P/N de cet antigène de référence était de l'ordre de 6-8. 24 sérums humains normaux non dilués ont été testés dans une réaction d'inhibition contre l'Ag de référence comparativement au PBS ; aucun d'entre eux n'a exercé d'action inhibitrice. Ultérieurement, 50 $\mu$l d'un mélange de ces sérums ont servi à établir la positivité à 100 % de la réaction. Les échantillons testés ont été considérés comme positifs lorsque l'inhibition était supérieure à 50 %.

* L'Ag NANB a été détecté dans les fèces de cinq patients souffrant d'hépatite NANB aiguë ; on n'a pas trouvé d'anticorps dans l'une d'entre elles. Chez deux autres patients, la maladie aiguë datait respectivement de 9 et de 3 mois et il n'a plus pu être détecté d'Ag dans les fèces. Le dernier des cinq patients avait présenté dans le passé une hépatite NANB avec de l'anticorps décelable qui a disparu au bout de 3 ans.

## TABLEAU 4

| Source de sérum⟍ Pays | Donneurs de sang | Personnel du Laboratoire | Patients en hémo-dialyse | Hépatite NANB sporadique |
|---|---|---|---|---|
| MAROC FRANCE | 8/78 0/26 | 0/12 | 1/12 | 19/28 7/8 |

Comme le montre le Tableau 4 ci-dessus, l'hémodialyse ou un travail de laboratoire impliquant la manipulation de sang humain ne paraît pas augmenter le risque d'infection par le virus NANB. Le fait que l'on utilise des Ac préparés à partir de singes infectés par un virus NANB épidémique, pour la détection d'Ag liés à un virus NANB sporadique, montre qu'il s'agit du même virus.

Les différentes étapes de l'étude qui viennent d'être décrites ont permis de caractériser un nouvel agent viral impliqué dans l'hépatite NANB. Cet agent viral semble être responsable aussi bien de la forme épidémique que de la forme sporadique et de la forme post-transfusionnelle de l'hépatite NANB.

Il est différent du virus HAV :

1) Une infection par le virus NANB épidémique a été observée chez un singe et chez des patients présentant une immunité anti-HAV ;

2) Les marqueurs usuels de l'infection aiguë (IgM anti-HAV) n'ont pas été détectés ;

3) Ce virus induit une réponse immune spécifique et les anticorps n'ont pas réagi avec une préparation virale de HAV ; des sérums de patients convalescents d'hépatite A ont donné des résultats négatifs dans le test de détection d'anticorps anti-virus NANB épidémique.

Le virus NANB épidémique est également distinct du HBV et par voie de conséquence du HDV :

1) La maladie a évolué chez certains patients présentant des anticorps anti-HBsAg et est également apparue chez un patient européen vacciné présentant une forte immunité anti-HBV ;

2) Il n'a généralement pas été détecté de HBsAg et HBeAg ; s'il préexiste des marqueurs de HBV à l'hépatite NANB (chez certains patients ivoiriens), il n'apparaît pas d'IgM anti-HBc ;

3) Des IgM de singes infectés par du virus NANB épidémique et utilisées pour la détection d'antigène viral, ne réagissent ni avec le HBV ou avec des préparations purifiées de HBsAg, ni avec des sérums de patients répliquant activement le HBV ; des anticorps anti-HBs n'interfèrent pas dans le test de détection d'antigène anti-virus NANB épidémique.

Le nouveau virus purifié caractérisé comme décrit dans ce qui précède semble pouvoir être isolé de sources largement réparties. En plus des cas décrits plus haut, l'antigène associé au virus a également été trouvé chez un patient sénégalais et des anticorps ont été trouvés dans le sérum d'un patient convalescent d'une épidémie ayant sévi en Algérie. L'agent NANB épidémique est considéré comme étant un virus véhiculé par l'eau. Néanmoins son rôle dans l'hépatite sporadique, en particulier dans un pays bénéficiant de bonnes conditions d'hygiène, n'exclut pas d'autres voies de transmission, et notamment la voie sanguine.

Au demeurant, l'implication du virus NANB épidémique dans des cas d'hépatite NANB sporadique ou post-transfusionnelle, devrait entraîner une reconsidération du qualificatif "épidémique".

Il résulte en effet des travaux de l'Inventeur que le virus identifié conformément à la présente invention, appelé virus de l'hépatite NANB épidémique, sporadique et transfusionnelle (en abrégé : "H-SET") est également responsable de l'apparition d'hépatite de type non A non B chez des malades transfusés.

Une étude comparative comprenant des malades polytransfusés sans hépatite infectieuse et des malades atteints d'hépatite A ou B a montré l'absence d'antigène associé au virus dans leurs selles et l'absence de virus cultivables dans les conditions dans lesquelles le virus "H-SET" se multiplie. La souche "H-SET" I-617 isolée des selles d'un malade souffrant d'hépatite NANB transfusionnelle, a pu être cultivée sur cellules PLC/PRP5 dans les conditions décrites plus haut. Les lésions correspondant notamment à de nombreuses formations syncitiales apparaissent à J6-J7 précédant le décollement des cellules infectées. Un procédé identique de culture est susceptible d'être utilisé pour un isolement du virus à partir d'un prélèvement sanguin. Les cultures infectées peuvent être utilisées comme source d'antigène non purifié en vue de la caractérisation à leur surface des molécules d'anticorps provenant d'un sujet ayant contracté l'infection (le complexe virus-anticorps étant révélé à l'aide d'anti-immunoglobulines humaines marquées).

La souche Clamart s'est avérée infectante pour deux singes rhésus (inoculation par ingestion (per os) avec 0,5 ml d'un extrait de selle à 10 %) avec apparition de l'antigène dans les selles entre les 20e et 24e jours après l'inoculation et avec augmentation des transaminases entre le 28e et le 32e jours (signe caractéristique d'atteinte hépatique).

**Revendications**

**Revendications pour les Etats contractants suivants : AT, BE, CH, DE, FR, GB, IT, LI, LU, NL, SE**

**1.** Agent viral purifié, associé à l'hépatite virale NANB sporadique, épidémique et post-transfusionnelle, caractérisé en ce qu'il est susceptible d'être obtenu à partir de fèces de patients atteints d'hépatique NANB et qu'il se présente sous la forme de particules virales sphériques de deux types principaux, associées à un même antigène viral :

a) des particules sphériques d'environ 75 nm de diamètre, dont la densité sur un gradient de CsCl est comprise entre 1,28 et 1,32 $g/cm^3$, et qui sédimentent dans les fractions d'un gradient de saccharose contenant entre 40 et 41,5 % (p/v) de saccharose,

b) des particules sphériques de 25 à 30 nm de diamètre, dont la densité sur un gradient de CsCl est comprise entre 1,12 et 1,19 g/cm$^3$, et qui sédimentent dans les fractions d'un gradient de saccharose contenant entre 26 et 28 % (p/v) de saccharose.

2. Souche d'un agent viral purifié selon la Revendication 1, dite "souche Clamart", déposée le 30 Octobre 1986 sous le numéro I-617, auprès de la Collection Nationale de Cultures de Microorganismes.

3. Souche d'un agent viral purifié selon la Revendication 1, dite "souche Ivoirienne", déposée le 30 Octobre 1986 sous le numéro I-616 auprès de la Collection Nationale de Cultures de Microorganismes.

4. Souche d'un agent viral purifié selon la Revendication 1, dite "souche H-SET", déposée le 5 Février 1987 sous le numéro 87.02.05.02 auprès de la Collection ECACC (Grande Bretagne).

5. Procédé d'isolement d'un agent viral selon l'une quelconque des Revendications 1 à 4, caractérisé en ce que l'on procède à la centrifugation, sur un gradient de CsCl ou sur un gradient de saccharose, d'un extrait biologique contenant ledit agent viral, et en ce qu'on recueille ledit agent dans les fractions du gradient de CsCl dont la densité est comprise entre 1,29 et 1,32 g/cm$^3$ ou dans les fractions du gradient de saccharose contenant entre 40 et 41,5% (p/v) de saccharose, sous forme de particules sphériques de 75 nm de diamètre environ, et/ou dans les fractions du gradient de CsCl dont la densité est comprise entre 1,12 et 1,19 g/cm$^3$, ou dans les fractions du gradient de saccharose contenant entre 26 et 28% (p/v) de saccharose, sous forme de particules sphériques de 25 à 30 nm de diamètre.

6. Procédé selon la Revendication 5, caractérisé en ce que l'extrait biologique comprenant l'agent viral est constitué par des fèces de patients atteints d'hépatite NANB.

7. Procédé selon la Revendication 5, caractérisé en ce que l'extrait biologique contenant l'agent viral est constitué par une culture du virus de l'hépatite NANB, sur une lignée cellulaire continue d'hépatocytes.

8. Procédé selon l'une quelconque des Revendications 6 ou 7, caractérisé en ce que l'extrait biologique est préalablement soumis à une centrifugation à 5000-8000 g, à l'issue de laquelle le surnageant est filtré à travers un filtre de porosité 0,22 $\mu$m.

9. Procédé selon la Revendication 8, caractérisé en ce que préalablement à la filtration et à la centrifugation, l'extrait biologique est soumis à un traitement de dissociation physique, notamment par ultra-sons, ou à un traitement de dissociation chimique.

10. Procédé de culture d'un agent viral selon l'une quelconque des Revendications 1 à 4, caractérisé en ce que des hépatocytes, ou des lignées cellulaires continues d'hépatocytes, sont inoculés par un extrait de fèces d'un patient atteint d'hépatite NANB, ou par un extrait de foie d'un singe inoculé par un extrait de fèces de patients souffrant d'hépatite NANB, et cultivés jusqu'à confluence de la culture.

11. Application d'un agent viral selon l'une quelconque des Revendications 1 à 4, au diagnostic in vitro de l'hépatite NANB épidémique, sporadique, et post transfusionnelle.

12. Procédé de détection d'anticorps spécifiques de l'hépatite NANB et associés à la fois à l'hépatite épidémique, sporadique, et post transfusionnelle, dans un échantillon de sang ou de produit dérivé du sang, lequel procédé est caractérisé en ce que dans une première étape, l'on met en contact ledit échantillon avec un réactif constitué par un agent viral selon l'une quelconque des Revendications 1 à 4, et dans une deuxième étape, on met en évidence la formation d'un complexe antigène-anticorps entre ledit agent viral, et des anticorps spécifiques de l'hépatite NANB éventuellement présents dans ledit échantillon.

13. Réactifs de diagnostic de l'hépatite virale NANB épidémique, sporadique ou post-transfusionnelle, caractérisés en ce qu'ils comprennent une préparation de l'antigène viral selon l'une quelconque des Revendications 1 à 4.

14. Agent thérapeutique pour le traitement de l'hépatite virale NANB épidémique, sporadique ou post-transfusionnelle, caractérisé en ce qu'il comprend essentiellement une préparation d'un agent viral

EP 0 277 437 B1

selon l'une quelconque des Revendications 1 à 4.

**15.** Réactifs de diagnostic de l'hépatite virale NANB épidémique, sporadique ou post-transfusionnelle, caractérisés en ce qu'ils comprennent des anticorps dirigés contre une préparation de l'antigène viral selon l'une quelconque des Revendications 1 à 4.

**16.** Agent thérapeutique pour le traitement de l'hépatite virale NANB épidémique, sporadique ou post-transfusionnelle, caractérisé en ce qu'il comprend des anticorps dirigés contre une préparation de l'antigène viral selon l'une quelconque des Revendications 1 à 4.

**Revendications pour les Etats contractants suivants : ES, GR**

**1.** Procédé d'isolement d'un agent viral associé à l'hépatite virale NANB sporadique, épidémique et post-transfusionnelle et susceptible d'être obtenu à partir des fèces de patients atteints d'hépatite NANB, caractérisé en ce que l'on procède à la centrifugation, sur un gradient de CsCl ou sur un gradient de saccharose, d'un extrait biologique contenant ledit agent viral, et en ce qu'on recueille ledit agent dans les fractions du gradient de CsCl dont la densité est comprise entre 1,29 et 1,32 g/cm$^3$, ou dans les fractions du gradient de saccharose contenant entre 40 et 41,5 % (p/v) de saccharose, sous forme de particules sphériques de 75 nm de diamètre environ, et/ou dans les fractions du gradient de CsCl dont la densité est comprise entre 1,12 et 1,19 g/cm$^3$, ou dans les fractions du gradient de saccharose contenant entre 26 et 28 % (p/v) de saccharose, sous forme de particules sphériques de 25 à 30 nm de diamètre, les deux types de particules étant associés à un même antigène viral :
a) des particules sphériques d'environ 75 nm de diamètre, dont la densité sur un gradient de CsCl est comprise entre 1,28 et 1,32 g/cm$^3$, et qui sédimentent dans les fractions d'un gradient de saccharose contenant entre 40 et 41,5 % (p/v) de saccharose,
b) des particules sphériques de 25 à 30 nm de diamètre, dont la densité sur un gradient de CsCl est comprise entre 1,12 et 1,19 g/cm$^3$, et qui sédimentent dans les fractions d'un gradient de saccharose contenant entre 26 et 28 % (p/v) de saccharose.

**2.** Procédé selon la Revendication 1, caractérisé en ce que l'on purifie un agent viral ayant les caractéristiques d'une souche dite "souche Clamart", déposée le 30 Octobre 1986 sous le numéro I-617, auprès de la Collection Nationale de Cultures de Microorganismes.

**3.** Procédé selon la Revendication 1, caractérisé en ce que l'on purifie un agent viral ayant les caractéristiques d'une souche, dite "souche Ivoirienne", déposée le 30 Octobre 1986 sous le numéro I-616 auprès de la Collection Nationale de Cultures de Microorganismes.

**4.** Procédé selon la Revendication 1, caractérisé en ce que l'on purifie un agent viral ayant les caractéristiques d'une souche, dite "souche H-SET", déposée le 5 Février 1987 sous le numéro 87.02.05.02 auprès de la Collection ECACC (Grande Bretagne).

**5.** Procédé selon la Revendication 1, caractérisé en ce que l'extrait biologique comprenant l'agent viral est constitué par des fèces de patients atteints d'hépatite NANB.

**6.** Procédé selon la Revendication 1, caractérisé en ce que l'extrait biologique contenant l'agent viral est constitué par une culture du virus de l'hépatite NANB, sur une lignée cellulaire continue d'hépatocytes.

**7.** Procédé selon l'une quelconque des Revendications 5 ou 6, caractérisé en ce que l'extrait biologique est préalablement soumis à une centrifugation à 5000-8000 g, à l'issue de laquelle le surnageant est filtré à travers un filtre de porosité 0,22 $\mu$m.

**8.** Procédé selon la Revendication 7, caractérisé en ce que préalablement à la filtration et à la centrifugation, l'extrait biologique est soumis à un traitement de dissociation physique, notamment par ultra-sons, ou à un traitement de dissociation chimique.

**9.** Procédé de culture d'un agent viral obtenu par un procédé selon l'une quelconque des Revendications 1 à 4, caractérisé en ce que des hépatocytes, ou des lignées cellulaires continues d'hépatocytes, sont inoculés par un extrait de fèces d'un patient atteint d'hépatite NANB, ou par un extrait de foie d'un

14

singe inoculé par un extrait de fèces de patients souffrant d'hépatite NANB, et cultivés jusqu'à confluence de la culture.

10. Application d'un agent viral obtenu par un procédé selon l'une quelconque des Revendications 1 à 4, au diagnostic in vitro de l'hépatite NANB épidémique, sporadique, et post transfusionnelle.

11. Procédé de détection d'anticorps spécifiques de l'hépatite NANB et associés à la fois à l'hépatite épidémique, sporadique, et post transfusionnelle, dans un échantillon de sang ou de produit dérivé du sang, lequel procédé est caractérisé en ce que dans une première étape, l'on met en contact ledit échantillon avec un réactif constitué par un agent viral obtenu par un procédé, selon l'une quelconque des Revendications 1 à 4, et dans une deuxième étape, on met en évidence la formation d'un complexe antigène-anticorps entre ledit agent viral, et des anticorps spécifiques de l'hépatite NANB éventuellement présents dans ledit échantillon.

12. Application d'un agent viral obtenu par un procédé selon l'une quelconque des Revendications 1 à 4 pour l'obtention d'un agent thérapeutique pour le traitement de l'hépatite virale NANB épidémique, sporadique ou post-transfusionnelle.

**Claims**

**Claims for the following Contracting States : AT, BE, CH, DE, FR, GB, IT, LI, LU, NL, SE**

1. Purified viral agent associated with sporadic, epidemic and posttransfusional viral hepatitis NANB, characterised in that it can be obtained from the faeces of patients suffering from hepatitis NANB and in that it is in the form of two main types of spherical viral particles associated with the same viral antigen:
   a) spherical particles of about 75 nm in diameter whose density in a CsCl gradient is between 1.28 and 1.32 $g/cm^3$ and which sediment in sucrose gradient fractions containing between 40 and 41.5% (w/v) of sucrose,
   b) spherical particles from 25 to 30 nm in diameter whose density in a CsCl gradient is between 1.12 and 1.19 $g/cm^3$ and which sediment in sucrose gradient fractions containing between 26 and 28% (w/v) of sucrose.

2. Strain of a purified viral agent according to Claim 1, called "Clamart strain", deposited with the Collection Nationale de Cultures de Microorganismes on 30 October 1986 under the number I-617.

3. Strain of a purified viral agent according to Claim 1, called "Ivoirian strain", deposited with the Collection Nationale de Cultures de Microorganismes on 30 October 1986 under the number I-616.

4. Strain of a purified viral agent according to Claim 1, called "H-SET strain", deposited with the ECACC Collection (Great Britain) on 5 February 1987 under the number 87.02.05.02.

5. Process for isolating a viral agent according to any one of Claims 1 to 4, characterised in that a biological extract containing the said viral agent is centrifuged in a CsCl gradient or in a sucrose gradient, and in that the said agent is collected in the CsCl gradient fractions whose density is between 1.29 and 1.32 $g/cm^3$ or in the sucrose gradient fractions containing between 40 and 41.5% (w/v) of sucrose, in the form of spherical particles of about 75 nm in diameter, and/or in the CsCl gradient fractions whose density is between 1.12 and 1.19 $g/cm^3$, or in the sucrose gradient fractions containing between 26 and 28% (w/v) of sucrose, in the form of spherical particles from 25 to 30 nm in diameter.

6. Process according to Claim 5, characterised in that the biological extract containing the viral agent consists of faeces from patients suffering from hepatitis NANB.

7. Process according to Claim 5, characterised in that the biological extract containing the viral agent consists of a culture of hepatitis NANB virus on a continuous liver cell line.

8. Process according to either of Claims 6 and 7, characterised in that the biological extract is subjected beforehand to centrifugation at 5000-8000 g, at the end of which the supernatant is filtered through a filter with a porosity of 0.22 $\mu$m.

9. Process according to Claim 8, characterised in that prior to the filtration and the centrifugation, the biological extract is subjected to a physical dissociation treatment, especially by sonication, or to a chemical dissociation treatment.

10. Process for culturing a viral agent according to any one of Claims 1 to 4, characterised in that liver cells or continuous liver cell lines are inoculated with a faecal extract from a patient suffering from hepatitis NANB, or with a liver extract from a monkey inoculated with a faecal extract from patients suffering from hepatitis NANB, and cultured until the culture becomes confluent.

11. Application of a viral agent according to any one of Claims 1 to 4, in the in vitro diagnosis of epidemic, sporadic and posttransfusional hepatitis NANB.

12. Process for the detection of antibodies specific for hepatitis NANB and associated at the same time with epidemic, sporadic and posttransfusional hepatitis, in a blood sample or in a sample of product derived from blood, which process is characterised in that, in a first stage, the said sample is brought into contact with a reagent consisting of a viral agent according to any one of Claims 1 to 4 and, in a second stage, the formation of an antigen-antibody complex between the said viral agent and antibodies specific for hepatitis NANB, which may be present in the said sample, is detected.

13. Reagents for the diagnosis of epidemic, sporadic or posttransfusional viral hepatitis NANB, characterised in that they contain a preparation of the viral antigen according to any one of Claims 1 to 4.

14. Therapeutic agent for the treatment of epidemic, sporadic or posttransfusional viral hepatitis NANB, characterised in that it essentially contains a preparation of a viral agent according to any one of Claims 1 to 4.

15. Reagents for the diagnosis of epidemic, sporadic or posttransfusional viral hepatitis NANB, characterised in that they contain antibodies directed against a preparation of the viral antigen according to any one of Claims 1 to 4.

16. Therapeutic agent for the treatment of epidemic, sporadic or posttransfusional viral hepatitis NANB, characterised in that it contains antibodies directed against a preparation of the viral antigen according to any one of Claims 1 to 4.

**Claims for the following Contracting States : ES, GR**

1. Process for isolating of a viral agent associated with sporadic, epidemic and posttransfusional viral hepatitis NANB and capable of being obtained from the faeces of patients suffering from hepatitis NANB, characterised in that a biological extract containing the said viral agent is centrifuged in a CsCl gradient or in a sucrose gradient, and in that the said agent is collected in the CsCl gradient fractions whose density is between 1.29 and 1.32 $g/cm^3$ or in the sucrose gradient fractions containing between 40 and 41.5% (w/v) of sucrose, in the form of spherical particles of about 75 nm in diameter, and/or in the CsCl gradient fractions whose density is between 1.12 and 1.19 $g/cm^3$, or in the sucrose gradient fractions containing between 26 and 28% (w/v) of sucrose, in the form of spherical particles from 25 to 30 nm in diameter, both types of particles being associated with the same viral antigen:
   a) spherical particles of about 75 nm in diameter whose density in a CsCl gradient is between 1.28 and 1.32 $g/cm^3$ and which sediment in the sucrose gradient fractions containing between 40 and 41.5% (w/v) of sucrose,
   b) spherical particles from 25 to 30 nm in diameter whose density in a CsCl gradient is between 1.12 and 1.19 $g/cm^3$ and which sediment in the sucrose gradient fractions containing between 26 and 28% (w/v) of sucrose.

2. Process according to Claim 1, characterised in that a viral agent is purified which has the characteristics of a strain, called "Clamart strain", deposited with the Collection Nationale de Cultures de Microorganismes on 30 October 1986 under the number I-617.

3. Process according to Claim 1, characterised in that a viral agent is purified which has the characteristics of a strain, called "Ivoirian strain", deposited with the Collection Nationale de Cultures de Microorganismes on 30 October 1986 under the number I-616.

4. Process according to Claim 1, characterised in that a viral agent is purified which has the characteristics of a strain, called "H-SET strain", deposited with the ECACC Collection (Great Britain) on 5 February 1987 under the number 87.02.05.02.

5. Process according to Claim 1, characterised in that the biological extract containing the viral agent consists of faeces from patients suffering from hepatitis NANB.

6. Process according to Claim 1, characterised in that the biological extract containing the viral agent consists of a culture of hepatitis NANB virus on a continuous liver cell line.

7. Process according to either of Claims 5 and 6, characterised in that the biological extract is subjected beforehand to centrifugation at 5000-8000 g, at the end of which the supernatant is filtered through a filter with a porosity of 0.22 $\mu$m.

8. Process according to Claim 7, characterised in that prior to the filtration and the centrifugation, the biological extract is subjected to a physical dissociation treatment, especially by sonication, or to a chemical dissociation treatment.

9. Process for culturing a viral agent obtained by a process according to any one of Claims 1 to 4, characterised in that liver cells or continuous liver cell lines are inoculated with a faecal extract from a patient suffering from hepatitis NANB, or with a liver extract from a monkey inoculated with a faecal extract from patients suffering from hepatitis NANB, and cultured until the culture becomes confluent.

10. Application of a viral agent obtained by a process according to any one of Claims 1 to 4, in the in vitro diagnosis of epidemic, sporadic and posttransfusional hepatitis NANB.

11. Process for the detection of antibodies specific for hepatitis NANB and associated at the same time with epidemic, sporadic and posttransfusional hepatitis, in a blood sample or in a sample of product derived from blood, which process is characterised in that, in a first stage, the said sample is brought into contact with a reagent consisting of a viral agent obtained by a process, according to any one of Claims 1 to 4, and, in a second stage, the formation of an antigen-antibody complex between the said viral agent and antibodies specific for hepatitis NANB, which may be present in the said sample, is detected.

12. Application of a viral agent obtained by a process according to any one of Claims 1 to 4 in the production of a therapeutic agent for the treatment of epidemic, sporadic or posttransfusional viral hepatitis NANB.

**Patentansprüche**
**Patentansprüche für folgende Vertragsstaaten : AT, BE, CH, DE, FR, GB, IT, LI, LU, NL, SE**

1. Gereinigtes virales Mittel, assoziiert mit der sporadischen, epidemischen und posttransfusionellen viralen Hepatitis NANB, dadurch **gekennzeichnet,** daß es aus dem Stuhl von Patienten mit Hepatitis NANB erhalten werden kann und daß es in der Form von kugelförmigen viralen Teilchen von zwei Haupttypen, die mit einem gleichen viralen Antigen assoziiert sind, vorliegt:
(a) kugelförmige Teilchen mit einem Durchmesser von ungefähr 75 nm, deren Dichte auf einem CsCl-Gradienten zwischen 1,28 und 1,32 g/cm$^3$ liegt und die in den Fraktionen eines Saccharosegradienten, die zwischen 40 und 41,5 % (Gewicht/Vol.) Saccharose enthalten, sedimentieren,
(b) kugelförmige Teilchen mit einem Durchmesser von 25 bis 30 nm, deren Dichte in einem CsCl-Gradienten zwischen 1,12 und 1,19 g/cm$^3$ liegt und die in den Fraktionen eines Saccharosegradienten, die zwischen 26 und 28 % (Gewicht/Vol.) Saccharose enthalten, sedimentieren.

2. Stamm eines gereinigten viralen Mittels nach Anspruch 1 mit der Bezeichnung "Stamm Clamart", hinterlegt am 30. Oktober 1986 unter der Nummer I-617 bei der Collection Nationale de Cultures de Microorganismes.

3. Stamm eines gereinigten viralen Mittels nach Anspruch 1 mit der Bezeichnung "Stamm Ivoirienne", hinterlegt am 30. Oktober 1986 unter der Nummer I-616 bei der Collection Nationale de Cultures de Microorganismes.

4. Stamm eines gereinigten viralen Mittels nach Anspruch 1 mit der Bezeichnung "Stamm H-SET", hinterlegt am 5. Februar 1987 unter der Nummer 87.02.05.02 bei der Collection ECACC (Großbritannien).

5. Verfahren zur Isolierung eines viralen Mittels nach einem der Ansprüche 1 bis 4, dadurch **gekennzeichnet,** daß man in einem CsCl-Gradienten oder einem Saccharosegradienten einen biologischen Extrakt, der das virale Mittel enthält, zentrifugiert, das Mittel in den Fraktionen des CsCl-Gradienten, deren Dichte zwischen 1,29 und 1,32 $g/cm^3$ beträgt, oder in den Fraktionen des Saccharosegradienten, die zwischen 40 und 41,5 % (Gewicht/Vol.) Saccharose enthalten, in Form kugelförmiger Teilchen mit einem Durchmesser von etwa 75 nm gewinnt und/oder in den Fraktionen des CsCl-Gradienten, deren Dichte zwischen 1,12 und 1,19 $g/cm^3$ beträgt, oder in den Fraktionen des Saccharosegradienten, die zwischen 26 und 28 % (Gewicht/Vol.) Saccharose enthalten, in Form kugelförmiger Teilchen mit einem Durchmesser von 25 bis 30 nm gewinnt.

6. Verfahren nach Anspruch 5, dadurch **gekennzeichnet,** daß der biologische Extrakt, der das virale Mittel umfaßt, aus dem Stuhl von Patienten mit Hepatitis NANB besteht.

7. Verfahren nach Anspruch 5, dadurch **gekennzeichnet,** daß der biologische Extrakt, der das virale Mittel enthält, aus einer Kultur des Hepatitis-NANB-Virus auf einer kontinuierlichen Hepatozytenzelllinie besteht.

8. Verfahren nach einem der Ansprüche 6 oder 7, dadurch **gekennzeichnet,** daß der biologische Extrakt zuvor bei 5000 bis 8000 g zentrifugiert wird, wonach der Überstand über ein Filter mit einer Porengröße von 0,22 $\mu$m filtriert wird.

9. Verfahren nach Anspruch 8, dadurch **gekennzeichnet,** daß vor der Filtration und der Zentrifugation der biologische Extrakt einer physikalischen Dissoziationsbehandlung, insbesondere durch Ultraschall, oder einer chemischen Dissoziationsbehandlung unterworfen wird.

10. Verfahren zur Züchtung eines viralen Mittels nach einem der Ansprüche 1 bis 4, dadurch **gekennzeichnet,** daß Hepatozyten oder kontinuierliche Hepatozytenzelllinien mit einem Stuhlextrakt eines Patienten mit Hepatitis NANB oder mit einem Extrakt der Leber eines Affen, der mit einem Stuhlextrakt von Patienten, die an Hepatitis NANB leiden, inokuliert worden war, inokuliert werden und bis zum Zusammenfließen der Kultur gezüchtet werden.

11. Verwendung eines viralen Mittels nach einem der Ansprüche 1 bis 4 zur in-vitro-Diagnose der epidemischen, sporadischen und posttransfusionellen Hepatitis NANB.

12. Verfahren zur Detektion von spezifischen Hepatitis-NANB-Antikörpern, die gleichzeitig mit der epidemischen, sporadischen und posttransfusionellen Hepatitis in Zusammenhang stehen, in einer Blutprobe oder einem von Blut abgeleiteten Produkt, dadurch **gekennzeichnet,** daß man im ersten Schritt die Probe mit einem Reagenz, das aus einem viralen Mittel nach einem der Ansprüche 1 bis 4 besteht, in Kontakt bringt, und in einem zweiten Schritt die Bildung eines Antigen-Antikörper-Komplexes zwischen dem viralen Mittel und den spezifischen Hepatitis-NANB-Antikörpern, die gegebenenfalls in der Probe vorliegen, nachweist.

13. Reagenzien zur Diagnose der epidemischen, sporadischen und posttransfusionellen viralen Hepatitis NANB, dadurch **gekennzeichnet,** daß sie ein Präparat eines viralen Antigens nach einem der Ansprüche 1 bis 4 umfassen.

14. Therapeutisches Mittel zur Behandlung der epidemischen, sporadischen oder posttransfusionellen viralen Hepatitis NANB, dadurch **gekennzeichnet,** daß es im wesentlichen ein Präparat eines viralen Mittels nach einem der Ansprüche 1 bis 4 umfaßt.

**15.** Reagenzien zur Diagnose der epidemischen, sporadischen und posttransfusionellen viralen Hepatitis NANB, dadurch **gekennzeichnet,** daß sie Antikörper gegen ein Präparat eines viralen Antigens nach einem der Ansprüche 1 bis 4 umfassen.

**16.** Therapeutisches Mittel zur Behandlung der epidemischen, sporadischen oder posttransfusionellen viralen Hepatitis NANB, dadurch **gekennzeichnet,** daß es Antikörper gegen ein Präparat des viralen Antigens nach einem der Ansprüche 1 bis 4 umfaßt.

**Patentansprüche für folgende Vertragsstaaten : ES, GR**

**1.** Verfahren zur Isolierung eines viralen Mittels im Zusammenhang mit der sporadischen, epidemischen und posttransfusionellen viralen Hepatitis NANB, das aus dem Stuhl von Patienten mit Hepatitis NANB isoliert werden kann, dadurch **gekennzeichnet,** daß man auf einem CsCl-Gradienten oder einem Saccharosegradienten einen biologischen Extrakt, der das virale Mittel enthält, zentrifugiert und daß man das Mittel aus den Fraktionen des CsCl-Gradienten, deren Dichte zwischen 1,29 und 1,32 g/cm$^3$ beträgt, oder aus den Fraktionen des Saccharosegradienten, die 40 bis 41,5 % (Gewicht/Vol.) Saccharose enthalten, in Form von kugelförmigen Teilchen mit einem Durchmesser von etwa 75 nm gewinnt und/oder aus den Fraktionen des CsCl-Gradienten, deren Dichte zwischen 1,12 und 1,19 g/cm$^3$ beträgt, oder aus den Fraktionen des Saccharosegradienten, die zwischen 26 und 28 % (Gewicht/Vol.) Saccharose enthalten, in Form von kugelförmigen Teilchen mit einem Durchmesser von 25 bis 30 nm isoliert, wobei die beiden Teilchentypen mit einem gleichen viralen Antigen in Zusammenhang stehen:
(a) kugelförmige Teilchen mit einem Durchmesser von ungefähr 75 nm, deren Dichte auf einem CsCl-Gradienten zwischen 1,28 und 1,32 g/cm$^3$ liegt und die in den Fraktionen eines Saccharosegradienten, die zwischen 40 und 41,5 % (Gewicht/Vol.) Saccharose enthalten, sedimentieren,
(b) kugelförmige Teilchen mit einem Durchmesser von 25 bis 30 nm, deren Dichte in einem CsCl-Gradienten zwischen 1,12 und 1,19 g/cm$^3$ liegt und die in den Fraktionen eines Saccharosegradienten, die zwischen 26 und 28 % (Gewicht/Vol.) Saccharose enthalten, sedimentieren.

**2.** Verfahren nach Anspruch 1, dadurch **gekennzeichnet,** daß man ein virales Mittel reinigt, das die Eigenschaften eines Stammes mit der Bezeichnung "Stamm Clamart", hinterlegt am 30. Oktober 1986 unter der Nummer I-617 bei der Collection Nationale de Cultures de Microorganismes, besitzt.

**3.** Verfahren nach Anspruch 1, dadurch **gekennzeichnet,** daß man ein virales Mittel reinigt, das die Eigenschaften eines Stammes mit der Bezeichnung "Stamm Ivoirienne", hinterlegt am 30. Oktober 1986 unter der Nummer I-616 bei der Collection Nationale de Cultures de Microorganismes, besitzt.

**4.** Verfahren nach Anspruch 1, dadurch **gekennzeichnet,** daß man ein virales Mittel reinigt, das die Eigenschaften eines Stammes mit der Bezeichnung "Stamm H-SET", hinterlegt am 5. Februar 1987 unter der Nummer 87.02.05.02 bei der Collection ECACC (Großbritannien), besitzt.

**5.** Verfahren nach Anspruch 1, dadurch **gekennzeichnet,** daß der biologische Extrakt, der das virale Mittel enthält, aus dem Stuhl von Patienten mit Hepatitis NANB besteht.

**6.** Verfahren nach Anspruch 1, dadurch **gekennzeichnet,** daß der biologische Extrakt, der das virale Mittel enthält, aus einer Kultur des Hepatitis-NANB-Virus auf einer kontinuierlichen Hepatozytenzelllinie besteht.

**7.** Verfahren nach einem der Ansprüche 5 oder 6, dadurch **gekennzeichnet,** daß der biologische Extrakt zuvor bei 5000 bis 8000 g zentrifugiert wird, wonach der Überstand über ein Filter mit einer Porengröße von 0,22 μm filtriert wird.

**8.** Verfahren nach Anspruch 7, dadurch **gekennzeichnet,** daß vor der Filtration und der Zentrifugation der biologische Extrakt einer physikalischen Dissoziationsbehandlung, insbesondere durch Ultraschall, oder einer chemischen Dissoziationsbehandlung unterworfen wird.

**9.** Verfahren zur Züchtung eines viralen Mittels, erhalten nach einem Verfahren nach einem der Ansprüche 1 bis 4, dadurch **gekennzeichnet,** daß Hepatozyten oder kontinuierliche Hepatozytenzelllinien mit einem Stuhlextrakt eines Patienten mit Hepatitis NANB oder mit einem Extrakt der Leber eines Affen, der mit einem Stuhlextrakt von Patienten, die an Hepatitis NANB leiden, inokuliert worden war, inokuliert werden und bis zum Zusammenfließen der Kultur gezüchtet werden.

10. Verwendung eines nach einem Verfahren nach einem der Ansprüche 1 bis 4 erhaltenen viralen Mittels zur in-vitro-Diagnose der epidemischen, sporadischen und posttransfusionellen viralen Hepatitis NANB.

11. Verfahren zur Detektion von Antikörpern, die für die Hepatitis NANB spezifisch sind und die gleichzeitig mit der epidemischen, sporadischen und posttransfusionellen Hepatitis in Zusammenhang stehen, in einer Blutprobe oder einem von Blut abgeleiteten Produkt, dadurch **gekennzeichnet,** daß man in einem ersten Schritt die Probe mit einem Reagenz, das aus einem viralen Mittel, das nach einem Verfahren nach einem der Ansprüche 1 bis 4 erhalten worden ist, in Kontakt bringt, und in einem zweiten Schritt die Bildung eines Antigen-Antikörper-Komplexes zwischen dem viralen Mittel und den für die Hepatitis NANB spezifischen Antikörpern, die gegebenenfalls in der Probe vorliegen, nachweist.

12. Verwendung eines viralen Mittels, erhalten nach einem Verfahren nach einem der Ansprüche 1 bis 4, zum Erhalt eines therapeutischen Mittels zur Behandlung der epidemischen, sporadischen oder posttransfusionellen viralen Hepatitis NANB.

FIG.2

EP 0 277 437 B1

# FIG. 3

100nm

## FIG. 4

## FIG. 5